# EUROPEAN PATENT APPLICATION

(11) **EP 1 985 236 A1**
(43) Date of publication of application: **29.10.2008**
(21) Application number: 07714268.5
(22) Date of filing: 15.02.2007
(51) Int. Cl.: A61B 6/03, A61B 5/055

(54) **IMAGE DISPLAY DEVICE AND PROGRAM**

(30) Priority: 17.02.2006 JP 2006041392
(71) Applicant: Hitachi Medical Corporation, Tokyo 101-0021 (JP)
(72) Inventor: GOTO, Yoshihiro, Chiyoda-ku, Tokyo, 101-0021 (JP); KUSANO, Suzushi, Hitachi-shi Ibaraki, 317-0076 (JP)
(74) Representative: Strehl Schübel-Hopf & Partner
(86) International application number: PCT/JP2007/052738
(87) International publication number: WO 2007/094412

(57) **Abstract**

An image display device includes: an input unit (10) for selectively inputting desired image data from a plurality of images of an object captured by a medial image diagnosis device (2) ; a control unit (11) for extracting at least a fluid region of an organ containing air in the object among image data selectively inputted by the input unit (10) and generating a multi-dimensional image of the fluid region of the extracted organ; and a display unit (14) for displaying the multi-dimensional image of the fluid region of the organ generated by the control unit (11).

## Description

### Technical Field

The present invention is related to an image display device and program for visualizing a lesion such as mesothelioma which is spreading in a film thickness form on a wall part in an organ of an obj ect to be examined, by 3-dimensionally imaging at least fluid including air in the organ.

### Background Art

A conventional image display device constructs a 3-dimensional image of blood flow in a blood vessel from a medical image such as a tomographic image of an object acquired by a medical image diagnostic apparatus, and displays the constructed 3-dimensional image of the blood flow (for example, Patent Document 1).
Patent Document 1: JP-A-H8-154917

### Disclosure of the Invention

### Problems to be Solved

However, in Patent Document 1, 3-dimensional imaging of blood flow was possible to be implemented only because the blood flows consistently and unidirectionally keeping a constant quantity in the blood vessel, thus imaging of fluid that moves erratically such as air still remains as a problem to be solved.

The objective of the present invention is to provide an image display device and program capable of 3-dimensionally imaging at least fluid including air in an organ.

### Means to Solve the Problem

An image display device of the present invention comprises:
input means for selectively inputting desired image data from a plurality of image data of an object imaged by a medical image diagnostic apparatus;
control means for extracting a fluid region including air of an organ of the object from selectively inputted image data by the input means, and generating a multi-dimensional image of the extracted fluid region of the organ; and
display means for displaying a multi-dimensionally image of the fluid region of the organ generated by the control means.

An image display program of the present invention is to make a computer to execute the following functions as a program:
input function to selectively input desired image data from a plurality of image data of an object imaged by a medical image diagnostic apparatus (2);
control function to extract at least a fluid region including air of the organ of the object from selectively inputted image data, and to generate a multi-dimensional image of the extracted fluid region of the organ; and
display function to display a multi-dimensionally image of the fluid region of the organ generated by the control function.

### Effect of the Invention

In accordance with the present invention, it is possible to provide an image display device and program capable of 3-dimensionally imaging at least fluid including air in an organ.

### Brief Description of the Diagrams

Fig. 1 is a block diagram exemplifying a system configuration including an image display device of the present invention.
Fig. 2 is a flow chart showing an operation example of the system in Fig. 1.
Fig. 3 shows a first subroutine in Fig. 1.
Fig. 4 (A) is a display example of the images of lung apex and lung base related to the process in Fig. 3.
Fig. 4 (B) shows an image display example of deleting mediastinal portions in the process of Fig. 3.
Fig. 4 (C) shows an image display example of half-open display in the process of Fig. 3.
Fig. 4 (D) shows images of lung apex and lung base using the central projective method in the process of Fig. 3.
Fig. 4 (E) shows the relationship among the viewpoint position, viewpoint direction and projection plane of the lung apex image and lung base image in the process of Fig. 3.
Fig. 5 shows a second subroutine in Fig. 1.
Fig. 6 (A) shows a display example of the process in Fig. 5.
Fig. 6 (B) shows another image display example of Fig. 6(A).
Fig. 6 (C) shows another display example different from Fig. 6 (A) and Fig. 6 (B).
Fig. 7 shows a third subroutine in Fig. 1.
Fig. 8 (A) shows a display example of the process in Fig. 7.
Fig. 8 (B) shows another image display example of Fig. 8(A).
Fig. 9 shows a fourth subroutine in Fig. 1.
Fig. 10 (A) shows a display example of the process in Fig. 9.
Fig. 10 (B) shows another image display example of Fig. 10 (A).
Fig. 10 (C) shows another image display example of Fig. 10 (A) and Fig. 10 (B).

### Description of the Symbols

2... medical image diagnostic apparatus, 10... input unit (mouse, keyboard), 11... control unit (CPU), 14... display unit (monitor)

### Best Mode to Carry Out the Invention

Hereinafter, the best mode to carry out the present invention will be described using the attached diagrams. In all of the diagrams to illustrate the embodiment of the present invention, the same symbols are appended to the parts having the same function, and the repeated description thereof will be omitted.

### [System Configuration]

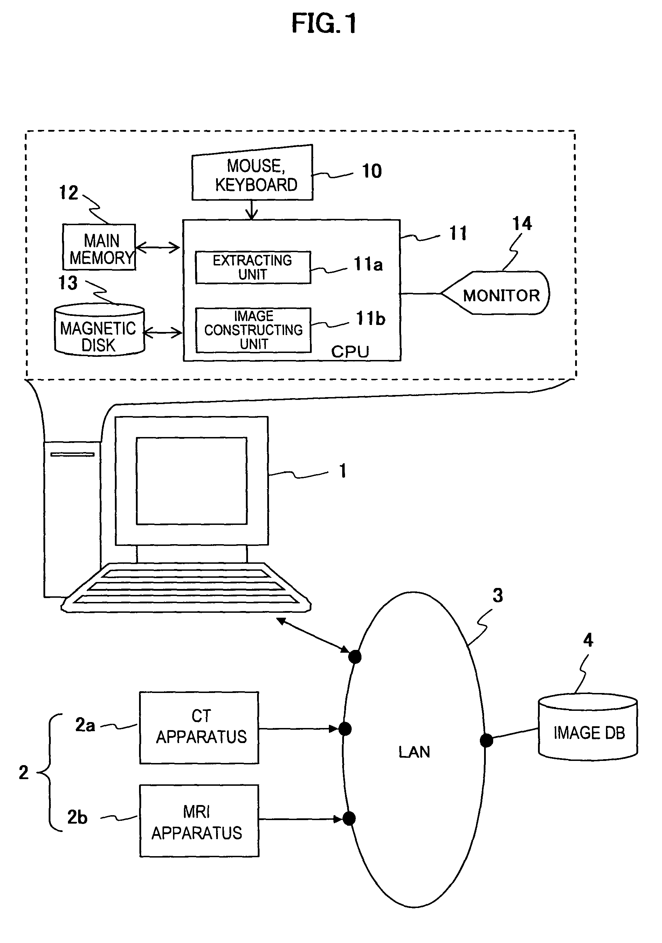

Fig. 1 is a hardware configuration diagram showing the system including the image display device and the surrounding devices thereof related to the present embodiment.

This system is configured by a medical imaging device 2 being connected by an image display apparatus 1 and a local area network (LAN) 3, and image database (DB) 4. The image display device 1 has an input unit 10 [input], control unit (CPU) 11 connected to the input unit 10, a main memory 12, magnetic disk 13 [control], and display unit (monitor) 14 [output].

### [Input]

The input unit 10 is a commonly known input device such as a mouse or keyboard, and an operator sets and inputs operation condition of the image display device or image data of the processing target. In concrete terms, the input unit 10 selectively inputs desired image data from a plurality of image data of the object, being imaged by a medical imaging apparatus 2.

### [Control]

The main memory 12 reads out image data of the processing target, and program for image processing or display processing from the magnetic disk 13. CPU 11 is for making the main memory 12 to execute programs for image processing or display processing under previously mentioned operation condition, with respect to image data of the processing target selectively inputted by the input unit 10, and has an extracting unit 11a and an image constructing unit 11b. The magnetic disk 13 stores a plurality of medical images including imaging data of a processing target and program for a variety of image or display processing. Here, CPU 11 operates to extract the fluid region in the body of the object from the image data selectively inputted by the input unit 10, and to calculate the image of the extracted fluid region.

### [Output]

The monitor 14 displays image data of the processing target to which the image or display program is executed. In other words, it displays the fluid region image calculated by the CPU 11.

The medical imaging apparatus 2 images medical images including tomographic images or 3-dimensional images of the object. Here, the representative example of the medical imaging apparatus is set as CT apparatus 2a and magnetic resonance imaging apparatus 2b. The CT apparatus 2a reconstructs a tomographic image of the object using transmission X-ray data from manifold direction of the object. The MRI apparatus 2b applies a gradient magnetic field to the object placed in a static magnetic field, and performs imaging of the information from inside of the object's body using magnetic nuclear resonance phenomenon of atomic elements contained in the object. The medical imaging apparatus does not have to be limited to the examples described hereto, but can include all kinds as long as capable of imaging tomographic images or 3-dimensional images of the object such as an ultrasonic diagnostic apparatus and nuclear medicine device.

The LAN 13 performs data transfer among the image display device 1, medical imaging apparatus 2 and an image database 4 which are connected thereto.
Image database 4 accumulates the medical images imaged by the medical imaging apparatus 2.

### [Main flow chart of the system operation]

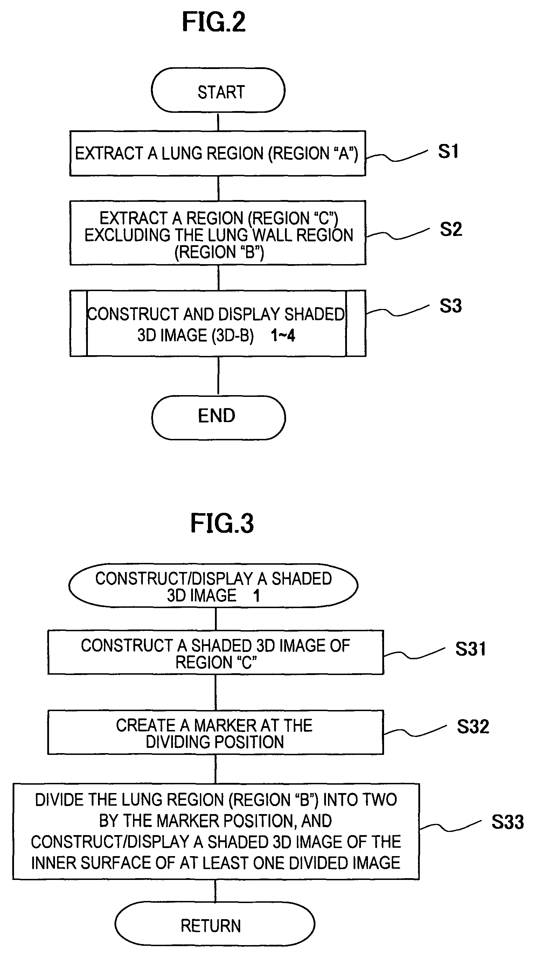

The process of the system operation example will now be described in accordance with Fig. 2. Fig. 2 is a main flow chart of the system operation. The processing below will be started after the CPU 11 reads the 3-dimensional image data of the lung of the object imaged by the medical imaging apparatus 2 or the image database 4 through operation by the operator using input unit 10. The operator may change the setting of the value of shading degree or opacity of the 3-dimensional image in advance before starting step S1. When the operator does not change the setting of these values, the values initialized in the image display apparatus 10 are to be used.

In the step S1, the lung region is extracted by the extracting unit 11a using means such as threshold processing. In the lung region, a tissue region and a lung cavity region are included. In the tissue region, the regions of the tissues such as a lung wall, mediastinum, bronchia tube or blood vessel are further included, and the regions such as a blood vessel or gas in the lung cavity region are included in the lung cavity region.

In step 52, the extracting unit 11a extracts image data of the tissue region from the 3-dimensional image data, and extracts image data of the lung cavity region by eliminating image data of the tissue region from image data of the lung region extracted in the step 1. In this way, by extracting a predetermined organ region and further extracting the region of the extracted predetermined organ (lung) excluding the wall region, at least the fluid region including air in the object can be extracted. Here, the fluid region indicates the air or gas region included in a lung, and the liquid secreted by the organ is also included in the air region.

In step 3, a 3-dimensional image (3D-A) of a gas region is calculated and displayed by shading using a method such as the surface rendering method, depth method or volume rendering (ray casting) method, based on the image data of the lung cavity region extracted by the image construction unit 11b in the step S2. Here, each embodiment is described in a form of using the sub-routine call method in the step S3.

### <First Embodiment>

A first embodiment calculates and displays the 3-dimensional image of the gas part of the fluid in the lung based on the 3-dimensional image data of which the lung of the object is imaged. Here, the embodiment will be described using Fig. 3 in a form that the lungs are divided into two by a marker 81.

In the step S31, a 3-dimensional image (3D-A) of the gas part is calculated and displayed, by shading on the basis of image data of the lung cavity region extracted by the image constructing unit 11b in the step S2. In other words, CPU 11 calculates the 3-dimensional image from the portion of image data which is selected and inputted from the fluid region in the object and performs shading process on the calculated 3-dimensional image, and the monitor 14 displays the shaded 3-dimensional image.

For example, in the case of using the surface rendering method, the image constructing unit 11b makes the contour line of the lung cavity region continuous, by embedding predetermined pixel values in the discontinuous points on a contour line thereof. As for the pixel value for embedding, the value higher than the pixel value (CT value) of the blood is preferable. The image constructing unit 11b accumulates a plurality of tomographic images to which the pixel values are embedded and forms a 3-dimensional image, and performs shading on the formed 3-dimensional image. In this way, the 3-dimensional image (3D-A) of the gas part of the lung cavity region can be calculated.

Also, in the case of using the volume rendering method, the image constructing unit 11b forms the 3-dimensional image by accumulating the images of the lung cavity region and performs shading on the formed 3-dimensional image, and the information of the blood vessel pattern inside of the lung will be added to the 3-dimensional image obtained by the surface rendering method.

Also, in the volume rendering method, the shading technique to make it look like as if the inside of the organ glows (luminescence 3D) may be used, which is disclosed in JP-A-2001-351120. This luminescence 3D is capable of highlighting the shaded cancer or the vicinity of the lung wall in high luminance, and facilitates the early discovery of disease such as mesothelioma caused by inhaling asbestos.

The image constructing unit 11b calculates the concave portion on the surface of the 3-dimensional image (3D-A) of the gas part. Here, for example, the method disclosed in JP-A-2002-325762 is used for calculating the concave portion. The image constructing unit 11b determines that there is a substance other than gas in the calculated concave portion, and based on the determination thereof changes the display mode of the calculated concave portion to the one different from the surface other than the concave portion of the 3-dimensional image. The different display modes here mean, for example, each of display color, display pattern and luminance, or the combination of these modes. The image constructing unit 11b superimposes and displays the 3-dimensioanl image (3D-A) of the gas part along with the 3-dimensional images of the parts other than gas that is changed to a different mode.

In a step 32, the marker 81 is displayed on the monitor 14 at the position to be divided on the 3-dimensioanl image (3D-A) using the input unit 10. The image constructing unit 11b displays a marker parallel to the selected direction on the 3-dimensional image (3D-A). In other words, CPU 11 calculates the marker indicating the dividing position of the organ region in the fluid region image, calculates the synthesized information of the calculated marker and the fluid region image, and the monitor 14 displays the calculated synthesized information. Then when the operator changes the setting of the marker 81 to a predetermined position on the display screen using the input unit 10, the setting of the dividing position on the 3-dimensional image (3D-A) of the gas part can be changed to an arbitrary position. That is, the input means 10 sets the marker out of the synthesized information displayed on the monitor 14 to an arbitrary position, CPU 11 calculates the fluid region image divided by the marker thereof as the divided fluid region image based on the position of the marker set by the input unit 10, and monitor 14 displays the divided fluid region image calculated by the CPU 11.

In a step S33, the image constructing unit 11b divides the image data of the lung wall region (tissue region) into two at the position of the marker. The image constructing unit 11b calculates the 3-dimensional image (3D-B) of the lung wall by shading the inner side of each (or one) of the divided lung walls, and displays the 3-dimensional image (3D-B) of the lung wall on the monitor 14. In other words, by generalizing the display thereof, the CPU 11 calculates the divided fluid region image from a plurality of viewpoint directions, and the monitor 14 displays the plurality of divided fluid region images calculated by the CPU 11 by juxtaposing or switching them.

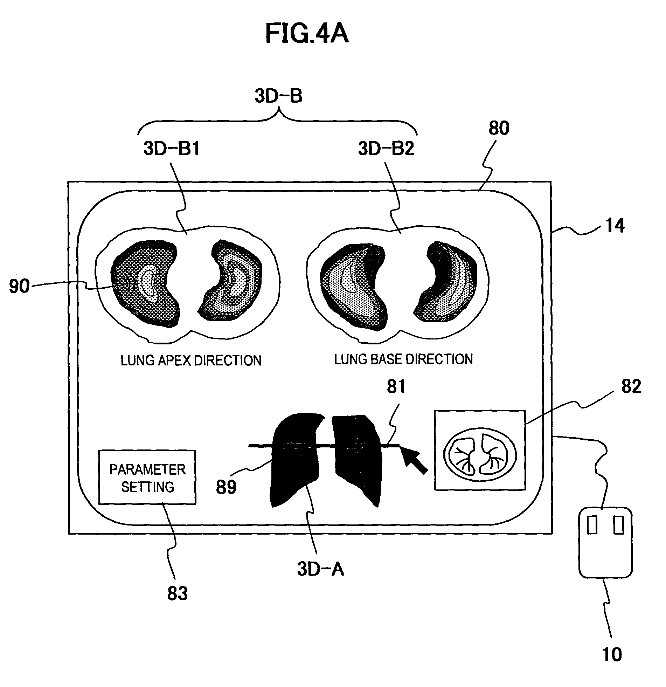

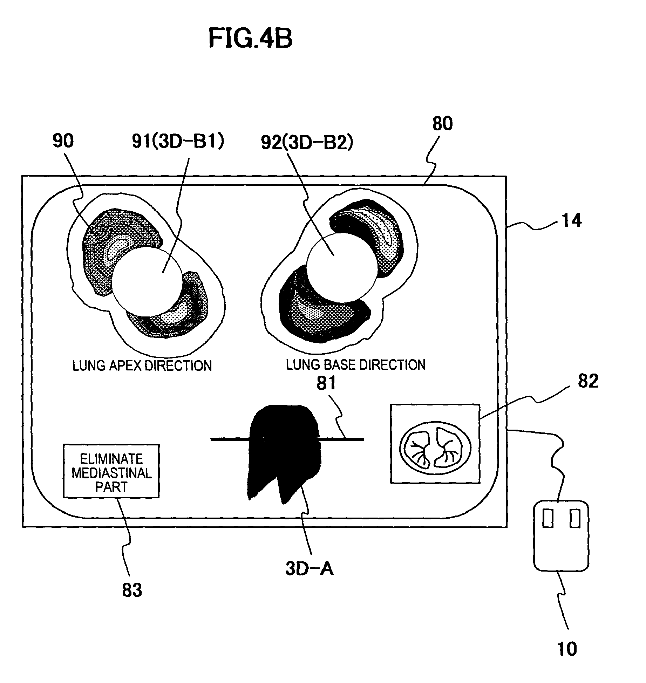

In Fig. 4A, an example is shown wherein the image constructing unit 11b sets the dividing position 81 as the direction parallel to the axial direction, and calculates a lung apex image (3D-B1) viewing the upper part of the lung from the set dividing position and a lung base image (3D-B2) viewing the bottom part from the set dividing position. Further, in this diagram, the marker 81 for indicating the dividing position for creating a lung wall image is superimposed and displayed on the 3-dimensional image of gas part (3D-A). Also, on the right side of the 3-dimensional image (3D-A) of gas, an axial image (tomographic image) 82 corresponding to the position of the marker 81 is displayed. On the lower left of the image display region of a screen 80, an icon 83 for "parameter setting" which is to set/change degree of shading or the value of opacity is displayed. Here, parameters to be set by the icon 83 are such as "projection method", "shading method", "diving direction setting", "elimination of mediastinal part" and "half-opened display". The "projection method" is for selecting parallel projection or central projection upon calculating a lung wall image. The "shading method" is for selecting a method such as depth, surface rendering method and volume rendering method for shading upon calculation of a 3-dimensional image of gas. The "setting dividing direction" is for selecting axial direction, sagittal direction or coronal direction as the dividing direction of the lung. The "elimination of mediastinal part" is, as shown in Fig. 4 B, for cutting out the region equivalent to the mediastinal part to make it not to be displayed, in the case that the mediastinal part which is located between the right and the left lungs blocks the lung wall upon interpretation of the image. In other words, generally the way to prevent the lung wall from being blocked would be that the input unit 10 sets the eliminating region which does not contribute pathologic diagnosis from the divided fluid region image displayed on the monitor 14, CPU 11 calculates the divided fluid region image excluding the eliminating region based on the eliminating region set by the input 10, and the monitor 14 displays the divided fluid region image excluding the eliminating region calculated by the CPU 11.

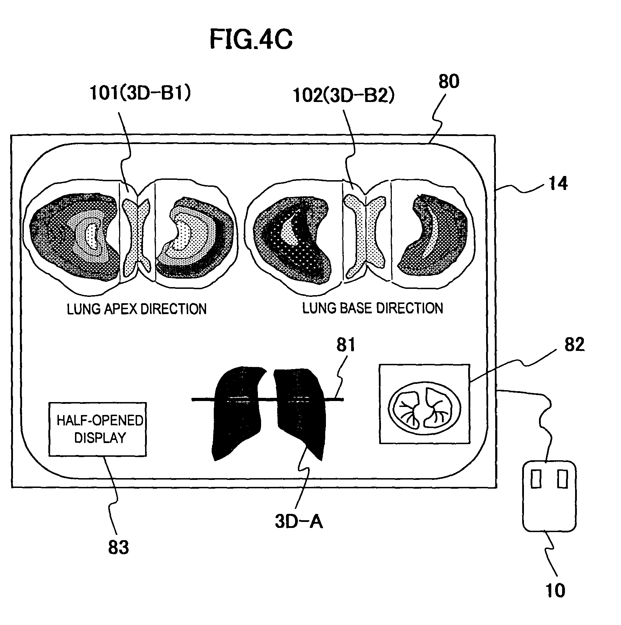

Also, the "half-opened display" is, as shown in Fig. 4C, for displaying the 3-dimensional lung wall image in a half-opened condition, and preventing the lung wall from being blocked by the mediastinal part. In other words, generalization of the "half-opened display" is that the input unit 10 sets the unfolding region on the divided fluid region image displayed on the monitor 14, the CPU 11 calculates the divided fluid region image wherein the unfolding region is unfolded based on the unfolding region set by the input 10, and the monitor 14 displays the divided fluid region image wherein the unfolded region calculated by the CPU 11 is being unfolded.

Next, the display mode of a screen 80 that is common in Figs. 4A ∼ 4C will be described. On the 3-dimensional image (3D-A) of the gas part in the screen 80, a concave portion 89 calculated by the image construction unit 11b is displayed by a display color different from the other surface. On the other hand, on a lung apex image (3D-B1), a convex portion 90 wherein the lung wall is raised is displayed. These concave portion 89 and convex portion 90 exist where they are engaged in the body of the object. In other words, the convex portion 89 and the concave portion 90 have a relationship that the concave portion 89 is formed as a result of the lung wall being raised and forming the convex portion 90 where the region should be filled with gas is intervened.

On the screen 80, the 3-dimensional image (3D-A) of the gas part inside of the lung is displayed. When existence of the concave portion and the position thereof are visually recognized upon observing the 3-dimensional image (3D-A) of the gas part, it can be presumed that there should be a convex portion (abnormal candidate shadow) on the wall corresponding to the position of the concave portion. Thus, this display mode facilitates the easier recognition of abnormality in the inner wall. Especially, by changing the display mode of the concave portion to the one different from the other surface, the concave portion is highlighted which can evoke the attention of an interpreter or operator.

While the procedure in the above embodiment of extracting the lung cavity region is to draw off the lung wall region extracted in the step S2 from the lung region extracted in the step 1, the method for extracting the lung cavity region does not have to be limited to this procedure. For example, the tissue region may be extracted first, and further inner region from the inner contour line of the tissue region by predetermined pixels may be extracted as the lung cavity region.

Fig. 4B is an image display example of the "elimination of the mediastinal part".

When the operator operates the icon 83 for the "elimination of the mediastinal part" using the input unit 10, the image construction unit 11b performs a mask processing on the vicinity of the mediastinum in the lung apex image (3D-B1) and the lung base image (3D-B2), and performs nondisplay processing on the mask-processed mediastinal part. The mediastinal region and the other region can be easily differentiated by using X-ray CT apparatus since the mediastinal region has larger absorbed amount of X-ray compared to the region filled with gas of the lung. Also, there is a method for executing the image processing regardless of an X-ray CT apparatus or MRI apparatus. The image constructing unit 11b measures the center of the gravity coordinate in the lung wall region of the right and the left lungs included in the lung apex image (3D-B1), and set the region of a predetermined length from the center of the gravity coordinate thereof as a mask region. By doing so, nondisplay processing can be performed on the mask-processed mediastinal parts as seen in regions 91 and 92. The same processing as the lung apex image is also performed on the lung base image (3D-B2). In this way, nondisplay processing is performed on mediastinal parts that are unnecessary for observing lung walls, which makes the observation the lung walls easier.

Fig. 4C is an image display example of the "half-opened display".

When the operator operates the "half-opened display" icon 83 using the input unit 10, the image constructing unit 11b cuts the image data of the lung wall (tissue region) near the center of the left and the right lungs in straight or curve line, and constructs a lung apex image (3D-B1) 101 and a lung base image (3D-B2) 102 wherein the portion near the marker 81 (view point position) is opened wider to the right and left side than the lung apex and the lung base (back side). The monitor 14 displays the constructed lung apex image (3D-B1) 101 and the lung base image (3D-B2) 102 which are opened to the left and the right. The lung apex image (3D-B1) 101 and the lung base image (3D-B2) 102 are the images being cut at the position of 3D-A in the body axis direction parallel to the axial cross-section. They are further cut in the vicinity of the mediastinal parts in the direction parallel to the sagittal direction, and the cut surfaces in the direction parallel to the sagittal direction are unfolded to the right and the left.

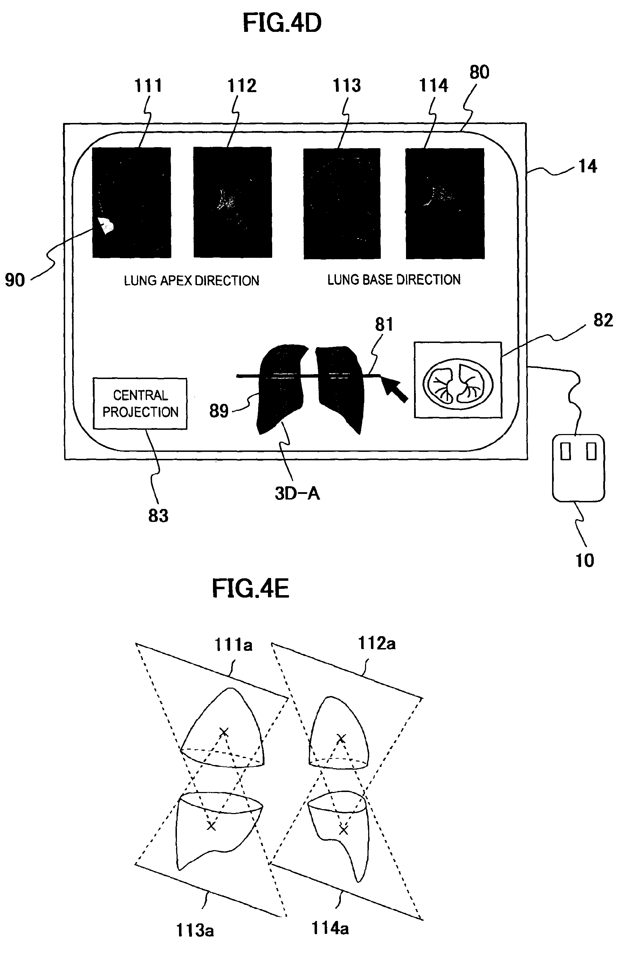

Fig. 4D is a pattern diagram showing an example of lung apex images 111, 112 and lung base images 113, 114 imaged by the central projection method. The image constructing unit 11b displays the inside of the lungs in wide range using the central projection method. Fig. 4E is a pattern diagram showing the positional relationship among the view point position, view point direction and the projection planes of the lung apex images 111, 112 and the lung base images 113, 114. The two lung apex images 111 and 112 are the images wherein the lung is cut in a direction parallel to the axial direction, the viewpoint positions are set in the lung fields on the lung base side, and the central projection is performed on the projection planes 111a and 112a viewing from the viewpoint positions toward the lung apex directions.

The two lung base images 113 and 114 are the images wherein the lung is cut in the direction parallel to the axial direction, the view point positions are set in the lung fields on the lung apex side, and the central projection is performed on the projection planes 113a and 114a from the viewpoint positions thereof toward the lung base direction. In other words, the input unit 10 sets the view points for performing the central projection with respect to the divided fluid region image displayed on the monitor 14, the CPU 11 calculates the central projection image based on the viewpoints set by the input unit 10, and the monitor 14 displays the central projection image calculated by the CPU 11.

Accordingly, by selecting the central projection or parallel projection as the method for calculating wall images, it is possible to provide the images based on the desired projection by the operator.

While the lung apex image and the lung base image are calculated as a pseudo 3-dimensional image in the aforementioned, they may be calculated as a 2-dimensional image, for example, a maximum value projection image.

Also, while the lung apex image and the lung base image are displayed as facing each other and the 3-dimensional image of the gas part is further juxtaposed in the aforementioned, it may be set so that one of the lung apex, lung base or the 3-dimensional image of the gas part is to be selected for display.

### [Effect of the present embodiment]

In the present embodiment, since the lung wall image is juxtaposed and displayed along with the 3-dimensioanl image of the gas part, when the concave portion 89 is found on the 3-dimensional image of the gas part, the convex portion 90 can be confirmed at once by referring to the lung wall image. Thus the lesion existing in the inner wall of a lung such as mesothelioma can be clearly visualized.

### <Second Embodiment>

The second embodiment calculates a ray-sum image (pseudo X-ray image) from the tomographic image in place of the 3-dimensional image of the gas in the first embodiment, and sets the dividing position on the calculated ray-sum image.

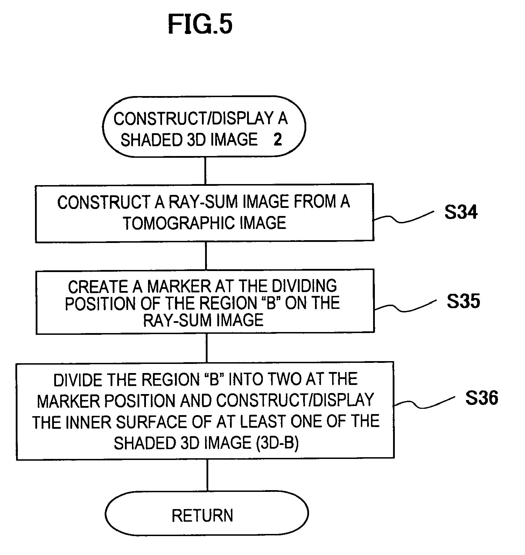

Flow of the processing in the second embodiment will be described in accordance with Fig. 5. Fig. 5 is a flow chart showing the flow of processing in the second embodiment.

In step S34, the image constructing unit 11b calculates a ray-sum image from the tomographic image in which the lung cavity region is extracted in S2. The ray-sum image is obtained by adding, for example, out of the tomographic image data loaded by the operator using the input unit 10, the pixel values which exist on the same virtual projection line, and diving the added value by the number of pieces of the added tomographic image. The monitor 14 displays the calculated ray-sum image. The step S35 may be processed in prior to the step S2 , or in parallel with the step S2.

In step S35, the marker 81 is displayed at the position to be divided on the ray-sum image using the input unit 10.

In step S36, the image constructing unit 11b calculates the 3-dimensional lung wall images (3D-B1, 3D-B2) wherein the image data (tissue region) of the lung wall region is divided into two at the marker position, as in the same manner in the step S32. The monitor 14 displays the calculated 3-dimensional images (3D-B1, 3D-B2). In other words, in accordance with the respective steps, the CPU 11 calculates a ray-sum image as the fluid image from the desired image data selected and inputted by the input unit 10, calculates the marker for dividing the calculated ray-sum image, further calculates the synthesized information of the calculated marker and the ray-sum image, and the monitor 14 displays the calculated synthesized information.

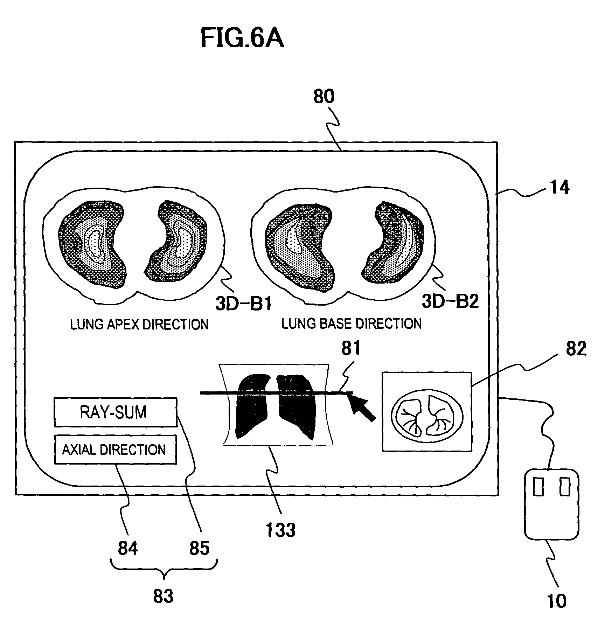

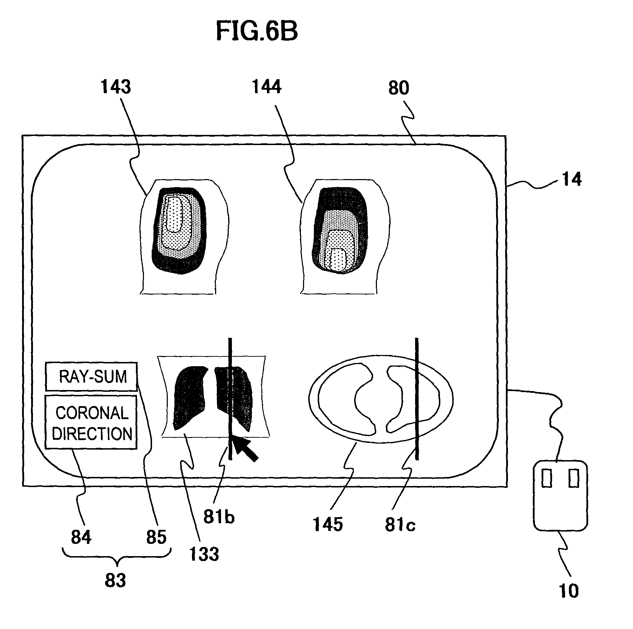

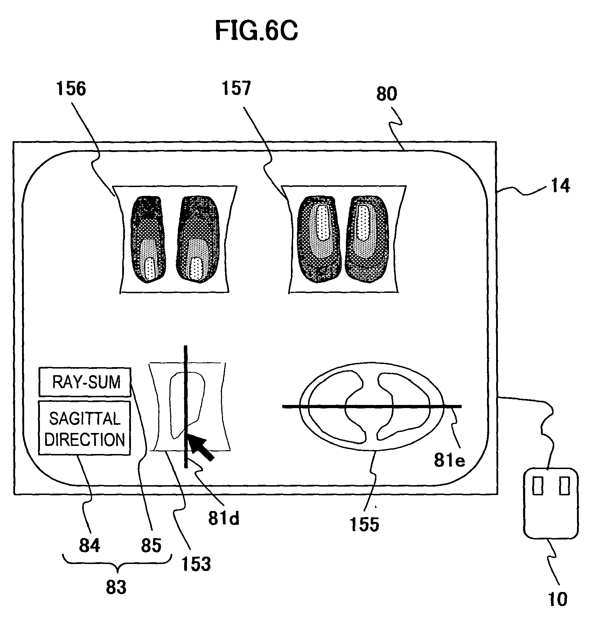

Fig. 6 is a pattern diagram showing an image display example displayed as a result of the above-described processing.

On a screen 80 in Fig. 6A, a ray-sum image 133 and the marker 81 that is parallel to the axial direction and is superimposed on the ray-sum image 133 are displayed. Also, an icon 83 for "parameter setting" displayed on the screen 80 is different in that an icon 84 for making the dividing direction of the marker axial direction and an icon 85 for denoting the ray-sum image are displayed, but other display is the same as the first embodiment.

Fig. 6B is a pattern diagram showing another image display example of Fig. 6A.

On a screen 80, the ray-sum image 133 and the marker 81b that is parallel to the sagittal direction and is superimposed on the ray-sum image 133 thereof are displayed. Also, on the screen 80, an axial image (virtual image) 145 for a marker to specify the dividing position is displayed, and a marker 81c is displayed on the axial image 145 for the marker at the position corresponding to the ray-sum image 133. The marker 81b on the ray-sum image 133 and the marker 81c on the axial image 145 for a marker can be variably selected by the input unit 10. When one marker is selected and moved on the screen 80, the other marker moves in parallel with the selected one. On the upper part of the screen 80, lung wall images 143 and 144 being divided at the position of the marker 81b are displayed. Here, the lung wall images 143 and 144 are the lung wall images of the left lung. The lung wall image 143 shows the image viewing from the position of the marker 81b toward the medistinal part direction, and the lung wall image 144 shows the image viewing from the position of the marker 81b toward the left edge side of the left lung. The icon displayed on the screen 80 is different from Fig. 6A in that the icon 84 id displayed for making the dividing direction of the marker coronal direction, but rest of the display is the same as Fig. 6A.

Fig. 6C is a pattern diagram showing another image display example different from Fig. 6A and Fig. 6B.

On the screen 80, the ray-sum image 153 and the marker 81d parallel to the sagittal direction that is superimposed on the ray-sum image 153 are displayed. Also, on the screen 80, an axial image (virtual image) 155 for a marker to specify the dividing position is displayed, and a marker 81e is displayed on the axial image 155 for the marker at the position corresponding to the ray-sum image 153. It is set so that when one of the marker 81d on the ray-sum image 153 or the marker 81e on the axial image 155 for the marker is moved using the input unit 10, the other one moves in parallel to the selected one. On the upper part of the screen 80, the lung wall images 156 and 157 are displayed upon being divided at the position of the marker 81d. The lung wall images 156 and 157 are the images of lung walls striding over the left and the right lungs, the lung wall image 156 is the image viewing from the position of the marker 81d toward the front of the left and the right lungs, and the lung wall image 157 is the image viewing from the position of the marker 81d toward the back of the left and the right lungs. The icon 83 for "parameter setting" is the same as Fig. 6A and Fig. 6B, except an icon 84 for making the dividing direction of the marker sagittal direction. In other words, the input unit 10 sets the marker out of the synthesized information displayed on the monitor 14 in the moving direction along at least one direction of axial direction, coronal direction or sagittal direction, CPU 11 updates and calculates the synthesized information with respect to the moving direction of the marker set by the input unit 10, and the monitor 14 displays the updated and calculated synthesized information.

While the ray-sum image is described in the above-embodiment, a maximum projection value image may be used in place of the ray-sum image.

### [The Effect of the Present Embodiment]

In accordance with the present embodiment, it is possible to determine the dividing position of a lung wall image, on the ray-sum image reflecting the air region and tissue region of the lung or the image for a marker.

In particular, ray-sum images can make the data quantity less than the 3-dimensional images and enable more doctors to interpret the image since it is closer to X-ray images, whereby excelling over the first embodiment as a method for finding mesothelioma during health check.

The maximum projection value image can also make the data quantity less than the 3-dimensional images, and projects only the maximum value of the tomographic images accumulated on the 3-dimensional space, whereby excelling over the first embodiment as a method for finding mesotheliam exists at the position hidden from the surface.

### <Third Embodiment>

The present embodiment is to rotatably move one of the images displayed in the first embodiment or the second embodiment, so as to make the other displayed image also rotatably move in parallel with it.

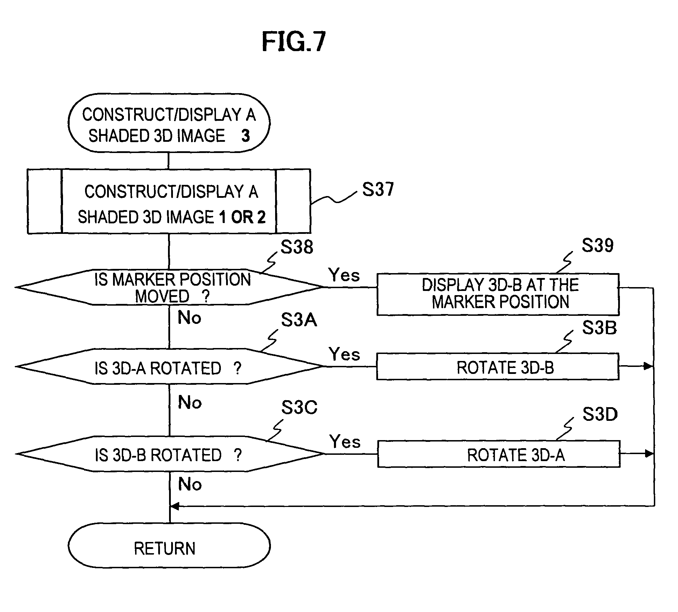

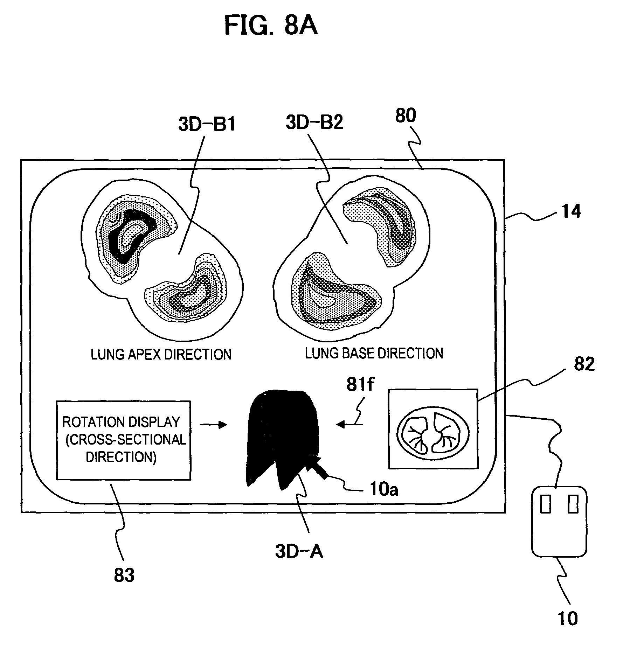

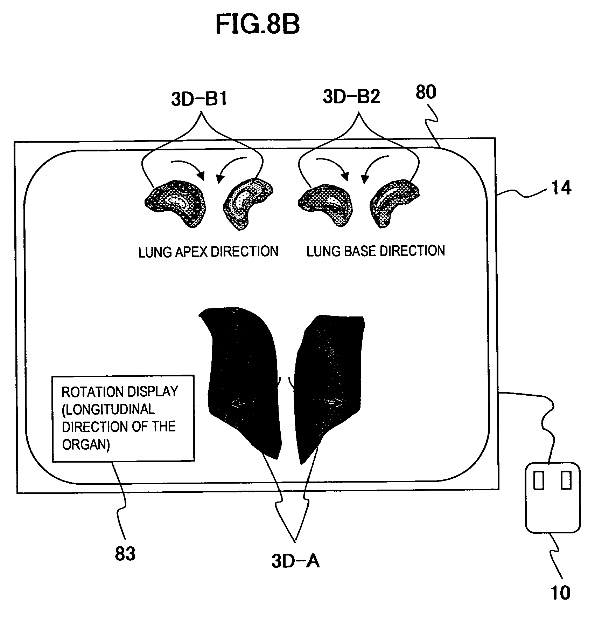

Fig. 7 is a flow chart showing the processing flow of the third embodiment, and Figs. 8A and 8B show an example of the display mode in Fig. 7.

In step S37, one of the images displayed in the first embodiment or the second embodiment is calculated and displayed. In concrete terms, as shown in Fig. 8A, a 3-dimensional image of a lung's gas part (3D-A), a lung apex image and a lung base image (3D-B) are displayed, and observation is started in the condition that the marker 81e denoting the dividing position of the lung apex image and the lung base image (3D-B1, 3D-B2) in the left and the right directions of the 3-dimensional image of the gas part (3D-A) is being displayed. On the screen 80, an axial image 82 corresponding to the marker 81e is also displayed.

In step S38, movement of the marker 81f is determined by the image constructing unit 11b. When the operator moves a cursor 10a of the input unit 10 to the marker 81f and further moves it in vertical direction of the screen 80, the image constructing unit 11b determines that the marker 81f is moved and proceeds with step S39. When the operator does not move the marker 81f, step S3A is to proceed. Also, image constructing unit 11b updates the axial image 82 to correspond to the position of the marker 81f.

In step S39, the image constructing unit 11b divides the lung wall image at the position of the marker 81f in the lung apex direction and the lung base direction, and calculates the lung apex image (3D-B1) and the lung base image (3D-B2) respectively. The monitor 14 displays the calculated lung apex image (3D-B1) and the lung base image (3D-B2) respectively. By doing so, the lung wall images (3D-B1) and (3D-B2) are respectively updated along with the movement of the marker 81f.

In step S3A, the image constructing unit 11b determines whether the 3-dimensional image (3D-A) of the gas part of the lung is rotated or not. When the operator operates the cursor 10a of the input unit 10 so as to rotatably move the 3-dimenisonal image (3D-A) of the gas part of the lung, the image constructing unit 11b detects the direction the rotation/movement of the 3-dimensional image (3D-A) of the gas part of the lung and proceeds to step S3B. When the operator does not rotatably move the 3-dimensional image (3D-A) of the gas part of the lung, step S3C is carried out.

In step 3B, the image constructing unit 11b rotates the lung apex image (3D-B1) and the lung base image (3D-B2) displayed on the monitor 14, in parallel to the rotation direction detected in the step S3A.

In the step S3C, the image constructing unit 11b determines whether the lung apex image (3D-B1) and the lung base image (3D-B2) are rotated or not. When the operator operates the cursor 10a of the input unit 10 so as to rotatably move the lung apex image (3D-B1) and the lung base image (3D-B2), the image constructing unit 11b detects the direction of the rotation movement of one of the lung apex image (3D-B1) or the lung base image (3D-B2) and proceeds with step S3D. When the operator does not rotatably move the lung apex image (3D-B1) and the lung base image (3D-B2), the operation returns to the main flow chart and the process is ended. When one of the lung apex image (3D-B1) and the lung base image (3D-B2) is rotatably moved, the other image is also rotatably moved in parallel with the one that was selected and moved.

In the step S3D, the image constructing unit 11b rotates the 3-dimensional image (3D-A) of the gas part of the lung displayed on the monitor 14 in parallel with the rotation direction detected in the step S3C.

Also, Fig. 8B is the pattern diagram showing another mode of the image display device capable of rotatably moving the displayed images.

On the screen 80, the lung apex image (3D-B1) of the gas part in the lung, the lung base image (3D-B2) that is the surface image and the icon 83 for indicating each process are displayed. In Fig. 8B, the left and the right lungs can be independently rotated with respect to the 3-dimensional image (3D-A) of the gas part of the lung. Fig. 8B shows the right lung rotated by 180° in a clockwise direction, and the left lung rotated by 180° in a counterclockwise direction. Then the image constructing unit 11b detects the rotation direction of the 3-dimensonal image of the respective gas parts of the right lung and the left lung, and rotatably moves the right lung of the lung apex image (3D-B1) and the right lung of the lung base image (3D-B2) concurrently by 180 in a clockwise direction, and the left lung of the lung apex image (3D-B1) and the left lung of the lung base image (3D-B2) concurrently in a counterclockwise direction. In other words, the input unit 10 sets the rotation direction on the shaded 3-dimensional image displayed on the monitor 14, CPU 11 calculates the rotated divided fluid region image with respect to the movement in the rotation direction set by the input unit 10, and the monitor 14 displays the divided fluid region image calculated by the CPU 11.

### [Effect of the Present Embodiment]

In accordance with the present embodiment, when the 3-dimensional image (3D-A) of the gas part of the lung is rotated, the lung apex image (3D-B1) and the lung base image (3D-B2) are also rotated in parallel with it. Thus, while recognizing the concave portion as rotating the 3-dimensional image (3D-A) of the gas part of the lung, the lung apex image (3D-B1) and the lung base image (3D-B2) of the same viewpoint direction can be observed, whereby improving the visibility for discovering a mesotheliam. Further, when the marker 81f is moved, the lung apex image (3D-B1), the lung base image (3D-B2) and the axial image 82 are updated corresponding to the position of the marker 81f. Therefore, for example, when the concave portion (the case for a suspect of mesotheliam) is found in the 3-dimensional image (3D-A) of the gas part of the lung, the marker 81f can be set at the position cutting across the concave portion so as to facilitate visual recognition of the axial image 82, the lung apex image (3D-B1) and the lung base image (3D-B2).

### <Fourth Embodiment>

A fourth embodiment is an image display device wherein the abnormality candidate shade detecting unit (CAD: Computer Aided Detection) function is added to the above respective embodiments. The image constructing unit 11b analyses the pixel value or depth of each pixel, determines the appropriateness of each condition based on the analysis result, sets the condition value based on the determination, and presents the existence of the set abnormal candidate shade to an interpreter. The image constructing unit 11b changes the display mode of the presented abnormal candidate shade to the one that is different from the one of normal regions other than the abnormal candidate region.

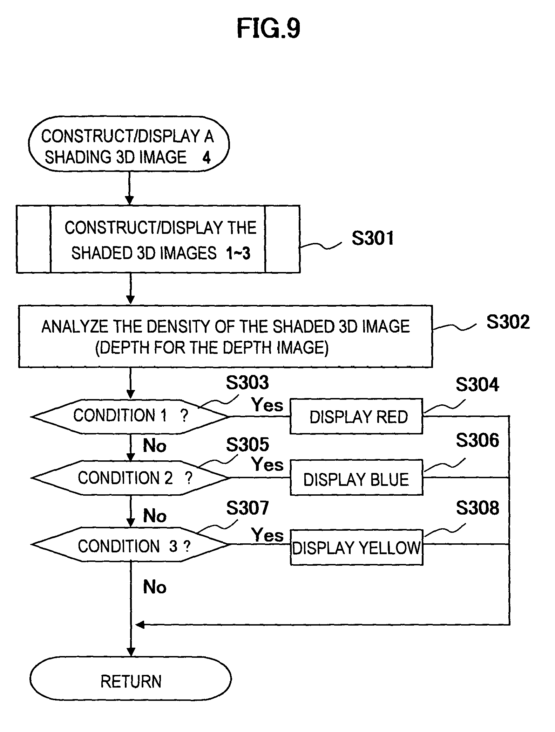

Fig. 9 is a flow chart showing the flow of the process in the fourth embodiment.

In step S301, one of the images displayed in the first, second or third embodiments is calculated and displayed. Here, the marker is displayed by a dotted line 81g since it can stand in the way of interpretation of the image after the setting of the line.

In step S302, the image constructing unit 11b analyses the density of each pixel of a 3-dimensional image of the gas part of the lung. When the 3-dimensional image of the gas part of the lung is constructed as a depth image, the depth is to be analyzed.

In step S303, the image constructing unit 11b determines whether the analyzed region satisfies the predetermined "condition 1" or not based on the density or depth of each pixel on the 3-dimenisonal image of the gas part of the lung. The "condition 1" is, for example, a condition such as "the density value is smaller than the surrounding region, the area of the low density region is smaller than the steady value, and the ratio between the major axis and the minor axis of the low density region is smaller than the steady value. When this condition is satisfied step S304 is carried out, and when the condition is not satisfied step S304 is carried out.

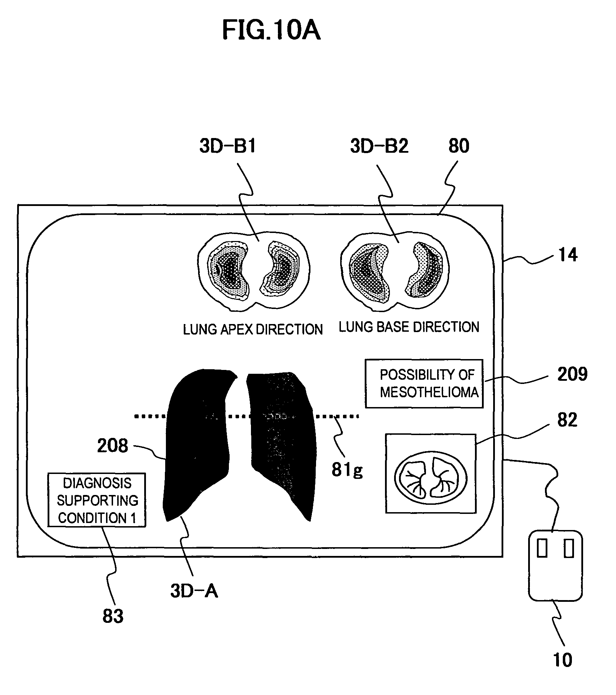

In step S304, the image constructing unit 11b displays in red color the pixels that are determined to satisfy the condition. Fig. 10A is an image display example wherein the pixels satisfying the condition 1 are displayed in red. In Fig. 10A, the 3-dimensional image (3D-A) of the gas part of the lung, the lung apex image (3D-B1) and the lung base image (3D-B2) which are the surface images, icon 83 for indicating each process, tomographic image 82 in the position of the marker 81f, and a message 209 for pointing out the possibility of illness due to inhaling asbestos are displayed. In the 3-dimensional image (3D-A) of the gas part of the lung, a portion 208 that satisfies the condition 1 is displayed in red.

In step S305, the image constructing unit 11b determines whether the region satisfies a "condition 2" or not based on the density or depth of each pixel on the 3-dimensional image of the gas part of the lung. The "condition 2" is, for example, that the density value is smaller than the surrounding region, an area S of the low density region is S1<S<S2, and a ratio R of the major axis/minor axis of the low density region is R1<R<R2. When this condition is satisfied step S306 is carried out, and if the condition is not satisfied step S307 is carried out.

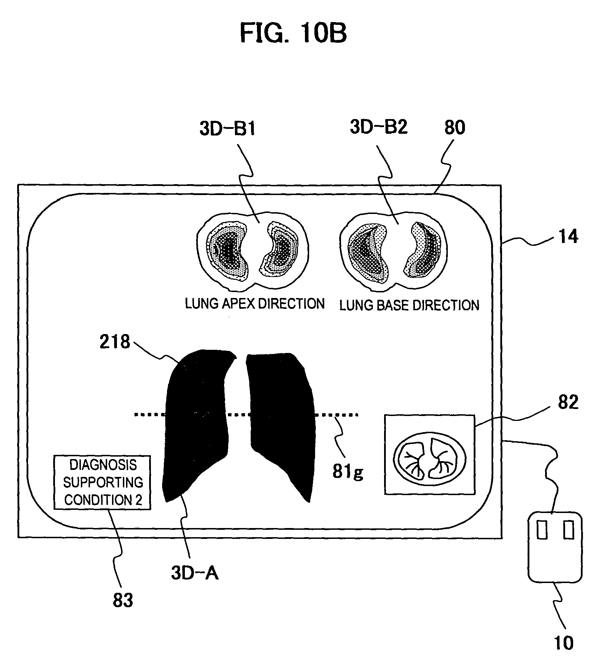

In step S306, the image constructing unit 11b displays in blue color the pixels that are determined as satisfying the condition. Fig. 10B is an image display example wherein the pixels which satisfy the condition 2 are displayed in blue. In Fig. 10B, a 3-dimensional image (3D-A) of the gas part of the lung, the lung apex image (3D-B1) and the lung base image (3D-B2) that are the surface images, the icon 83 for indicating each process and the tomographic image 82 at the position of the marker 81f are displayed. Out of the 3-dimensional image (3D-A) of the gas part of the lung, a portion 218 that satisfies the condition 2 is displayed in blue.

In step S307, the image constructing unit 11b determines whether the region satisfies "condition 3" or not based on the density or depth of each pixel on the 3-dimensional image of the gas part of the lung. The "condition 3" is, for example, that the density value is lower than the surrounding region, area S of the low density region is S4<S<S5, ratio R of the major axis/minor axis of the low density region is R4<R<R5, and CT1 which is the CT value of the place corresponding the original CT image is CT1<CT<CT2. When this condition is satisfied step S308 is carried out, and if the condition is not satisfied the process returns to the main flow to be ended.

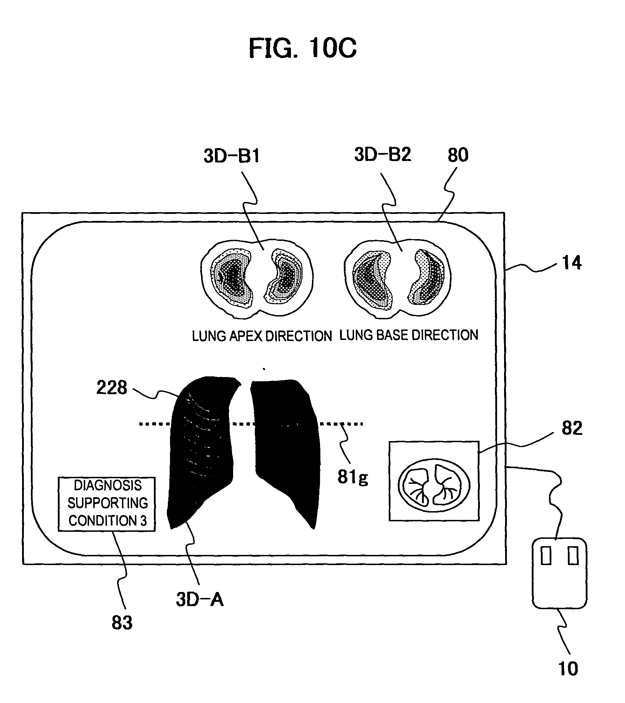

In step S308, the image constructing unit 11b, as shown in Fig. 10C, displays in yellow color a pixel 228 which is determined that satisfy the condition 3.

While the analysis on the 3-dimensional image is described in the present embodiment, a 2-dimensonal image (slice image) may be analyzed and the corresponding 3-dimensional image may be colored.

Also, while the conditions 1 - 3 are described in the present embodiment, the condition may be N-numbers and the colors for indicating the meeting of the respective conditions may be displayed.

Also, illness due to inhaling asbestos has characteristics of having a pattern of the concave portion appearing on the 3-dimensional image of the gas part of the lung being elongated, or appearing in radial pattern centering around a point when there are a number of them. Given this factor, as a condition for determining the illness by the image constructing unit 11b, "the ratio between the major axis and minor axis is more than 3" may be used for determining the shape of the concave portion, or as the condition for measuring the concentration ratio when there are a plurality of concave portions "the intersecting point of the straight lines or the strip-shaped regions wherein a predetermined width is given to the straight lines thereof which connect two points that are farthest from each other in the respective concave portions is within a predetermined range (within a predetermined pixel count)" may be used.

Then when a concave portion satisfying these conditions is found, the image constructing unit 11b determines that there is an illness candidate region due to inhaling asbestos. The monitor 14 displays the indication of the determined result such as "possible mesotheliam" on a display area 209. In this way, the display can attract more attention of the operator.

The operator may input the values for the users to arbitrarily set such as the ratio between the major axis and the minor axis, concentration degree and CT values using the input unit 10 to make the image constructing unit 11b to update the condition values for determining the illness.

Also, the image constructing unit 11b may be configured to obtain the correlation between the shape of the concave portion on the 3-dimensional image of the gas part of the lung and the rib shape of the object obtained from the image data of the object or the rib shape for the reference stored in a magnetic disk 13 in advance, using the pattern matching process.

Then when it is determined that the concave portion matches the rib shape, the image constructing unit 11b displays the concave portion thereof using the same display color as the region other than the concave region on the 3-dimensional image of the gas part of the lung. By doing so, it is possible to avoid lowering of visibility due to mix-up of the concave portion of the 3-dimensional image of the gas part of the lung influenced by the rib shape and the concave portion of the 3-dimensional image of the gas part of the lung due to the illness.

Fig. 10C is an image display example of a concave portion corresponding to a rib (displayed in dotted line) without coloring which is displayed with the same color as the other surface (normal portion) other than the concave portion thereof. In Fig. 10C, a 3-dimensional image (3D-A) of the gas part of the lung, the lung apex image (3D-B1) and the lung base image (3D-B2) as surface images, an icon 83 for indicating each process, and a tomographic image 82 at the position of a marker 81g are displayed. While the concave portion due to a rib bone is displayed on the 3-dimensional image (3D-A) of the gas part of the lung is displayed using a dotted line for illustrative purposes in Fig. 10C, this dotted line does not have to be displayed in the actual screen display. Also, the other surfaces other than the ribs are displayed in the same display mode as the lung.
In order to show the interpreter that the concave portion may be of the rib bone, a dotted line or other display mode may be used to display the concave portion. In sum, the CPU 11 analyses the density information of the shaded 3-dimensioanl image, and the monitor 14 displays the divided fluid region image from the density information of the 3-dimensional image analyzed by the CPU 11 using different colors.

### [Effect of the Present Embodiment]

In accordance with the present embodiment, it is possible to display the determination result of the abnormal shade candidate by changing the display colors on the 3-dimensional image of the gas part of the lung, whereby improving the convenience for image diagnosis.

### <Other Embodiments>

While the 3-dimensional image of the gas part wherein the air part in the lung is calculated based on the 3-dimenisonal image data of the object's lung in the above-mentioned embodiments, the fluid image does not have to be limited to the 3-dimensional image of the gas part of the lung, and any image of any region may be used as long as it is the fluid image of the region of the object filled with equable fluid. For example, a blood image wherein the blood image in circulation organs of the object may be calculated, or a 3-dimensional image of the gas part wherein the air in digestive organs, ears, nose and respiratory organs are imaged may be calculated.

For example, a blood image.wherein inside of a blood vessel is imaged may be calculated by performing the same process as the above-described embodiments based on the 3-dimenisnoal image wherein the blood vessel of the object is imaged. Also, a 3-dimensional image of the inner wall of the blood vessel may be calculated along with the blood image. There is a concave portion on the surface of the blood image. In this case, there is a high possibility that this concave portion is formed by the blood being pushed toward the central direction of the blood vessel because of the polyp which is formed on the inner wall of the blood vessel being imaged in both the 3-dimensional image and the inner wall image of the blood vessel. In this way, concave/convex condition formed in the inner wall of the blood vessel can be recognized just by observing the calculated blood image. This method has a potential for diagnosing the inner condition of the blood vessel without imaging with injection of contrast medium.

Also, a 3-dimenisonal image of the gas part in a stomach may be calculated by performing the same process as the above embodiments based on the 3-dimensional image of the object's stomach. On the surface of the 3-dimensional image of the gas part of a stomach, there are concave portions and convex portions having inverse pattern such as puberulent portion of the stomach wall image. The concave portion has high possibility that it is formed by the air being pushed toward inner direction of the stomach due to a polyp formed on the inner wall of the stomach that is imaged both in the 3-dimensional image and the stomach wall image. The convex portion is formed by air getting into the convex portion of the stomach wall due to an ulcer.

In this way, by calculating the 3-dimensional image of the gas part of a stomach, the concave and convex condition formed in the inner wall of the stomach can be visually recognized just by observing the image. This method has potential for visually recognizing the condition in a stomach without performing an X-ray fluoroscopic examination wherein the inner wall image of the stomach is imaged by taking barium contrast medium.

Further, as another embodiment, wall images such as a lung wall image, blood vessel wall image and inner wall image of a stomach may be calculated by setting the cutting direction in an arbitrary direction instead of axial direction, sagittal direction, coronal direction or parallel direction.

Also, while a 3-dimensional image is described above for the image to be constructed, this invention may be applied to a 2-dimensional image clearly presenting the air part indicating the lumen of the extracted organ or a so-called 4-dimensional image wherein a plurality of 3-dimensional images are obtained and displayed with motion.

The above-described embodiments can obtain the function, method and result of the respective embodiments by making a computer to execute the following functions as a program:
an input function for selectively inputing desired image data from a plurality of image data of an object imaged by a medical image diagnostic apparatus (2);
a control function for extracting a fluid region of the object from the selectively inputted image data, and calculating the image of the extracted fluid region; and
a display function for displaying the calculated fluid image.

## Claims

1. An image display device comprising:
input means for selectively inputting desired image data from a plurality of image data of an object to be examined being imaged by a medical image diagnostic apparatus;
control means for extracting at least a fluid region including air of an organ of the object from the image data selectively inputted by the input means, and constructing a multiple-dimensional image of the fluid region of the extracted organ; and
display means for displaying a multi-dimensional image of the fluid region of the organ constructed by the control means.

2. The image display device according to claim 1, wherein the control means has:
a first extracting means for extracting the organ region of the object from the image data selectively inputted by the input means;
a second extracting means for extracting the wall part from the extracted organ region; and
a third extracting region for extracting at least the fluid region including air of the organ based on the wall part of the extracted organ region.

3. The image display device according to claim 1, wherein the control means has image constructing means for preparing a fluid region of the extracted organ for the portion of the plurality of image data, and constructing a 3-dimensional image of the fluid region of the organ from the fluid region of the organ of the prepared plurality of images.

4. The image display device according to claims 1 ∼ 3, wherein:
the control means calculates a marker indicating the dividing position of the organ region in the calculated fluid region image, and calculates the synthesized information of the calculated marker and the fluid region image; and
the display means displays the synthesized information.

5. The image display device according to claim 4, wherein:
the input means sets the marker out of the synthesized information displayed on the display means to an arbitrary position;
the control means calculates the fluid region image divided by the marker thereof based on the position of the marker being set by the input means; and
the display means displays the divided fluid region image calculated by the control means.

6. The image display device according to claim 5, wherein:
the control means calculates the divided fluid region image from a plurality of viewpoint directions; and
the display means displays the plurality of divided fluid region images calculated by the control means by juxtaposing or switching them.

7. The image display device according to claim 5, wherein:
the input means sets an eliminating region which does not contribute to diagnosis of illness from the divided fluid region displayed on the display means;
the control means calculates the divided fluid region image excluding the eliminating region based on the eliminating region set by the input means; and
the display means displays the divided fluid region image excluding the eliminating region calculated by the control means.

8. The image display device according to claim 5, wherein:
the input means sets an unfolding region on the divided fluid region image displayed on the display means;
the control means calculates the divided fluid region image wherein the unfolding region wherein the unfolding region set by the input means is unfolded; and
the display means displays the divided fluid region image wherein the unfolding region which is calculated by the control means is unfolded.

9. The image display device according to claim 5, wherein:
the input means sets a viewpoint for performing central projection with respect to the divided fluid region image displayed on the display means;
the control means calculates a central projection image based on the viewpoint set by the input means; and
display means displays the central projection image calculated by the control means.

10. The image display device according to claims 1 ∼ 3, wherein:
the control means calculates a ray-sum image as the fluid image from desired image data selectively inputted by the input means, calculates a marker for dividing the calculated ray-sum image, and further calculates the synthesized information of the calculated marker and the ray-sum image; and
the display means displays the calculated synthesized information.

11. The image display device according to claim 10, wherein:
the input means sets the marker in the synthesized information displayed on the display means in the moving direction along at least one of axial direction, coronal direction and sagittal direction;
the control means updates and calculates the synthesized information with respect to the movement in the moving direction of the marker set by the input means; and
the display means displays the updated and calculated synthesized information.

12. The image display device according to claim 4, wherein:
the input means sets the rotating direction on the shaded 3-dimensional image displayed on the display means;
the control means calculates the divided fluid region image rotated with respect to the movement in the rotating direction set by the input means; and
the display means displays the divided fluid region image calculated by the control means.

13. The image display device according to claim 1, wherein:
the control means analyses density information of the shaded 3-dimensional image; and
the display means displays the divided fluid region image by colors based on the density information of the 3-dimensional image analyzed by the control means.

14. The image display device according to claim 1, wherein the control means has:
a first extracting means for extracting an organ region of the object from the image data selectively inputted by the input means;
a second extracting means for extracting the wall part of from the extracted organ region;
a third extracting means for extracting at least a fluid region including air of the organ based on the wall part of the extracted organ region; and
image constructing means for preparing the fluid region of the extracted organ for the plurality of image data, and constructs a 3-dimensional image of the fluid region of the organ from the plurality of prepared fluid regions of the organ in the images.

15. An image display program for making a computer execute the following functions as a program:
input function for selectively input a desired image data from a plurality of image data of the object being imaged by a medical image diagnostic apparatus;
control function for extracting at least a fluid region including air of the organ of the object from the selectively inputted image data, and constructing a multi-dimensional image of the extracted fluid region of the organ; and
display function for displaying the constructed multi-dimensional image of the fluid region of the organ.
